# EUROPÄISCHE PATENTSCHRIFT

(11) **EP 2 954 911 B1**
(45) Veröffentlichungstag und Bekanntmachung des Hinweises auf die Patenterteilung: **15.04.2020**
(21) Anmeldenummer: 15168735.7
(22) Anmeldetag: 21.05.2015
(51) Int. Cl.: A61M 1/16, A61M 1/34, A61M 1/36

(54) **DIALYSEMASCHINEN-INTEGRIERTE SUBSTITUATPUMPE**
INTEGRATED DIALYSIS MACHINES SUBSTITUTE PUMP
POMPE DE LIQUIDE DE SUBSTITUTION INTÉGRÉE DANS UNE MACHINE DE DIALYSE

(30) Priorität: 12.06.2014 DE 102014108227
(43) Veröffentlichungstag der Anmeldung: 16.12.2015
(73) Patentinhaber: B. Braun Avitum AG, 34212 Melsungen (DE)
(72) Erfinder: Wolff, Henrik, 37213 Witzenhausen (DE); Bröker, Björn, 34355 Staufenberg (DE)
(74) Vertreter: Winter, Brandl, Fürniss, Hübner, Röss, Kaiser, Polte - Partnerschaft mbB

(56) Entgegenhaltungen:
- EP-A1- 2 240 218
- EP-A1- 2 586 474
- EP-A1- 2 666 491
- EP-A1- 2 737 915
- WO-A1-2008/125893
- WO-A1-2013/017239
- WO-A1-2013/180154
- WO-A2-2012/010322
- DE-A1- 3 444 671
- DE-A1-102006 050 272

## Beschreibung

Die Erfindung betrifft eine Dialysemaschine mit einer Internfluidik, wobei die Internfluidik folgende Teile hat:
- eine Dialysierflüssigkeitspumpe, um frische Dialysierflüssigkeit von einem Dialysierflüssigkeitsreservoir durch eine Dialysierflüssigkeitszuleitung zu einem Dialysator und verbrauchte Dialysierflüssigkeit von dem Dialysator durch eine Dialysierflüssigkeitsableitung zu einem Abfallreservoir zu pumpen,
- eine Substituatpumpe bzw. Substitutionspumpe, um Dialysierflüssigkeit durch eine Substituatzuleitung bzw. Substitutionszuleitung als Substituat einem extrakorporalen Schlauchsystem mit patientenseitigen Anschlüssen zuzuführen und
- Kupplungseinrichtungen zum Anschließen des extrakorporalen Schlauchsystems an die Internfluidik.

Sie betrifft des Weiteren eine Dialysemaschine mit einer Internfluidik, wobei die Internfluidik folgende Teile hat:
- eine Zuleitung, um eine Flüssigkeit einem extrakorporalen Schlauchsystem mit patientenseitigen Anschlüssen zuzuleiten und
- eine Kupplungseinrichtung, die strömungstechnisch mit der Zuleitung verbunden ist, um eine Schlauchleitung des extrakorporalen Schlauchsystems mit der Zuleitung zu koppeln, welche Kupplungseinrichtung nutzerseitig zugänglich an einer Maschinenfront der Dialysemaschine angeordnet ist, wobei die Dialysemaschine eine Abflussleitung aufweist, um Flüssigkeit aus dem extrakorporalen Schlauchsystem abzuleiten.

Eine Dialysemaschine weist grundsätzlich zwei Flüssigkeitssysteme (Fluidkreisläufe) auf, das sogenannten extrakorporale Blutleitungssystem, das in der vorliegenden Anmeldung auch als Externfluidik bezeichnet wird, sowie das sogenannte Dialysierflüssigkeitssystem, auch als Internfluidik bezeichnet, das in der Maschine (als Maschinenbestandteil) angeordnet ist. Das extrakorporale Blutleitungssystem und die Internfluidik stehen über den Dialysator bzw. dessen Membran in (Stoffaustausch-) Verbindung. Das extrakorporale Blutleitungssystem wird über einen arteriellen und einen venösen Zugang mit dem intrakorporalen Blutkreislauf eines Patienten verbunden. Aus der Internfluidik kann physiologische Flüssigkeit, insbesondere Dialysierflüssigkeit, dem extrakorporalen Blutleitungssystem zugeführt werden, z.B. um hohe Flüssigkeitsmengen, die dem Patienten während einer Dialysebehandlung geplant entzogen werden, wieder zuzuführen.

Aus der EP 2 666 491 A1 ist ein Hämodialysegerät bekannt mit einer dauerhaften und desinfizierbaren Internfluidik und einer austauschbaren Wegwerf-Externfluidik, wobei die Externfluidik alle blutseitigen Leitungen und einen Dialysator aufweist, und die Interfluidik folgende Teile hat:
- eine Bilanzierpumpeneinrichtung, die aus einer Dialysierflüssigkeitspumpe und einer mit dieser gekoppelten Abfallpumpe gebildet ist, wobei die Dialysierflüssigkeitspumpe frische Dialysierflüssigkeit von einer Dialysierflüssigkeitsquelle zu dem Dialysator und die Abfallpumpe verbrauchte Dialysierflüssigkeit von dem Dialysator zu einem Abfallziel pumpt und
- eine separate Substituatpumpe, die frische Dialysierflüssigkeit von der Dialysierflüssigkeitsquelle zu der Externfluidik pumpt.

Zum Verbinden der Externfluidik mit der Internfluidik sind an der Bedienungsseite des Hämodialysegeräts eine Substituatkupplung (Substitutionsflüssigkeitskupplung) sowie Dialysierflüssigkeitskupplungen angeordnet.

Aus der DE 10 2012 004 970 A1 ist ein Verfahren zum Dosieren eines mittels einer Blutbehandlungsvorrichtung erzeugten Substituats sowie eine Blutbehandlungsvorrichtung zum Durchführen des Verfahrens bekannt. Bei dem Verfahren erfolgt eine Dosierung mittels einer Hydraulik oder eines Hydraulikabschnitts der Blutbehandlungsvorrichtung. Die Hydraulik weist wenigstens eine in eine Dialysierflüssigkeitskammer eines Blutfilters oder eines Dialysators mündende oder dem Dialysator Dialysierflüssigkeit zuführende Dialysierflüssigkeitszulaufleitung auf. Die Hydraulik weist ferner wenigstens eine Substituatleitung sowie eine erste Filterstufe und eine zweite Filterstufe auf.

Aus der DE 3 444 671 A1 ist ein Hämodiafiltrationsgerät mit einem geschlossenem Dialysierflüssigkeitskreislauf bekannt. Der Kreislauf weist ein Bilanziersystem auf, dessen erste Kammer zwischen Dialyisierflüssigkeitsversorgung und Dialysator liegt und dessen zweite Kammer sich zwischen Dialysator und Abfluss erstreckt. Wobei von dem Dialysierflüssigkeitsweg zwischen der ersten Kammer und dem Dialysator eine Ultrafiltrationsvorrichtung abzweigt die mit dem Blutweg verbunden ist und wenigstens einen Sterilfilter und eine Pumpe aufweist.

Aus der DE 10 2006 050 272 A1 ist ein Hämodialysegerät, ein Hämodiafiltrationsgerät, ein Verfahren zur Probennahme bei solchen Geräten und ein Probenentnahmeset zur Anwendung bei solchen Geräten und Verfahren bekannt. Bei dem Hämodialyse- oder Hämodiafiltrationsgerät können bereits vorhandene Anschlüsse mit einem Kurzschlusstück verbunden werden um eine Reinigung zu erleichtern oder mit einem Probenentnahmeset verbunden werden, zur Bereitstellung von Substitutionsflüssigkeit.

Aus der WO 2013/017239 A1 ist ein Verfahren zum Entfernen von Fluid aus einem zur Blutbehandlung eines Patienten verwendeten Blutfilter und/oder zum Entfernen von Blut aus einem extrakorporalen Blutkreislauf durch aktives oder passives Einbringen von Luft oder einer Flüssigkeit in die Dialysatzulaufleitung nach Beendigung der Blutbehandlungssitzung bekannt. Ferner wird eine medizinische Behandlungsvorrichtung mit einer Steuerungs- und/oder Regelungsvorrichtung offenbart, mit der das Verfahren durchführbar ist.

Aus der EP 2 586 474 ist eine Hämodialysevorrichtung bekannt und insbesondere eine Hämodialysevorrichtung bei der eine Anreicherungsleitung die Dialysierflüssigkeitsversorgungsleitung mit dem Blutkreislauf kommuniziert um frische Dialysierflüssigkeit in den Patienten zurück zu transferieren.

Aus der WO 2012/010322 ist ein Konnektor, eine medizintechnische Behandlungsvorrichtung, eine medizintechnische Funktionseinrichtung sowie ein Verfahren zum Verbinden einer medizinischen Behandlungseinrichtung mit einer medizinischen Funktionseinrichtung bekannt. Der Konnektor ist geeignet zum Herstellen einer Fluidverbindung zwischen wenigstens zwei medizintechnischen Systemen, aufweisend einen mehrlumigen Abschnitt.

Aus der EP 2 240 218 A0 ist eine Vorrichtung zur Infusion eines Substitutionsmittels durch eine Dialysemaschine bekannt. Die Vorrichtung enthält eine Spüleinheit, die intern eine Spülkammer definiert, einen Verbindungskegel, der sich in dieser Kammer erstreckt und eine Verbindungseinheit die angepasst ist den Verbindungskegel mit einem Kanal zu verbinden um das Substitutionsmittel in den Patienten geben.

Aus der EP 2 737 915 A1 ist eine Dialyseextraktionsvorrichtung bekannt, die eine Dialyseextraktionseinheit mit einem Einlass und einem Auslass aufweist, die eine Flüssigkeit zirkulieren lassen kann und die ferner einen Sammelschnittstelle aufweist die die zirkulierende Flüssigkeit sammeln kann. Des Weiteren weist die Vorrichtung eine Abdeckung für die Sammelschnittstelle auf, die die Sammelschnittstelle ein und ausschaltet.

Aus der WO 2013/180154 A1 ist ein Hämodialysevorrichtung bekannt mit einem Dialysator, einem Blutkreislauf und einem Dialysierflüssigkeitskreislauf. Wobei eine Ersatzlösungsschnittstelle in dem Dialysierflüssigkeitskreislauf bereitgestellt wird und durch eine Abdeckung geöffnet oder geschlossen werden kann und somit den Dialysierflüssigkeitskreislauf mit dem Blutkreislauf verbinden kann.

Bei bekannten Blutbehandlungsvorrichtungen ist es in der Regel von Nachteil, dass bei einem Wechsel zwischen verschiedenen Therapieformen, beispielsweise Hämodialyse (HD), Hämofiltration (HF) oder Hämodiafiltration (HDF), unterschiedliche Externfluidiken, auch als extrakorporale Blut- oder Schlauchsysteme bezeichnet, an die Maschine anzuschließen sind. Dabei ist allein schon die Vorhaltung der verschiedenen Schlauchsysteme aufwändig und kostenintensiv. Des Weiteren birgt ein Wechseln oder auch ein Ergänzen der Externfluidik die Problematik, dass Sterilität und Dichtigkeit gewährleistet sein muss. Insbesondere können infolge eines Wechsels der Externfluidik zusätzliche Verfahrensroutinen notwendig sein, wie zum Beispiel ein Priming (Spülen des Dialysator zu Therapiebeginn), ein Freispülen des Dialysators während einer Therapie, ein Befüllen des extrakorporalen Schlauchsystems mit Patientenblut oder ein Reinfundieren von Patientenblut nach einer Therapie. Insgesamt verursacht jede Veränderung des extrakorporalen Schlauchsystems einen relativ hohen Aufwand.

Ausgehend von dem vorstehend beschriebenen Stand der Technik liegt der vorliegenden Erfindung die Aufgabe zugrunde, die zuvor angeführten Nachteile zu beseitigen, insbesondere Dialyseteilprozesse, vor allem solche, die eine physiologische Flüssigkeit erfordern, zu optimieren. Die Eignung der Dialysemaschine zur Reinigung und Desinfektion soll vorzugsweise verbessert werden. Weiterhin sollen mit der Dialysemaschine vorzugsweise verschiedene Blutbehandlungen durchzuführen sein, wobei der Aufwand bei einem Wechsel der Blutbehandlung gering und die Dialysemaschine möglichst einfach zu handhaben sein sollte.

Die Erfindung wird durch die Merkmale des unabhängigen Anspruch 1 definiert.

Nach einem ersten Aspekt der vorliegenden Erfindung wird diese Aufgabe gelöst durch eine Dialysemaschine mit einer Internfluidik gemäß vorstehender Definition, wobei die Internfluidik folgende Teile/Elemente hat:
- eine Dialysierflüssigkeitspumpe, die dafür angepasst ist, Dialysierflüssigkeit von einem Dialysierflüssigkeitsreservoir durch eine Dialysierflüssigkeitszuleitung zu einem Dialysator und von dem Dialysator durch eine Dialysierflüssigkeitsableitung zu einem Abfallreservoir zu pumpen,
- eine Substituatpumpe oder Substituatfördereinrichtung, die dafür angepasst ist, Dialysierflüssigkeit durch eine Substituatzuleitung (als Substituat) einem extrakorporalen Blutleitungssystem/Schlauchsystem mit patientenseitigen Anschlüssen zuzuführen und
- Kupplungseinrichtungen, die dafür angepasst sind, das extrakorporale Schlauchsystem an die Internfluidik anzuschließen.

Erfindungsgemäß weist die Internfluidik, die im Wesentlichen aus zwei Abflussleitungen besteht, der Dialysemaschine eine Abflussleitung auf, die von zwei der Kupplungseinrichtungen, die zum Anschließen des extrakorporalen Schlauchsystems angepasst sind, zu einem Abflussleitungsabschnitt führen in dem sie sich vereinen und von dort zu der Dialysierflüssigkeitsableitung und zum Abfallreservoir oder Abfall-Entsorgungsleitungund zwei Substituatzuleitungen mit jeweils einer Kupplungseinrichtung zum Anschließen des extrakorporalen Blutleitungssystems .

Bei der Abflussleitung handelt es sich um eine gesonderte oder separate Leitung, die zusätzlich zur Dialysierflüssigkeitsableitung vorgesehen ist. Diese gesonderte Leitung weist an ihrem einen Ende eine eigene Kupplungseinrichtung auf und führt von dieser direkt oder indirekt zum Abfallreservoir. Anders ausgedrückt ist die Abflussleitung strömungstechnisch direkt oder indirekt mit dem Abfallreservoir verbunden. Die Dialysierflüssigkeitsableitung weist ebenfalls eine eigene Kupplungseinrichtung auf und führt ebenfalls direkt oder indirekt zum Abfallreservoir. An der erfindungsgemäßen Dialysemaschine sind daher wenigstens zwei Kupplungseinrichtungen vorgesehen, über die durch einen Nutzer eine Verbindung vom extrakorporalen Schlauchsystem zum Abfallreservoir eingerichtet werden kann. Die Dialysierflüssigkeitspumpe kann insbesondere durch eine Dialysierflüssigkeitszuleitungspumpe und eine Dialysierflüssigkeitsableitungspumpe realisiert sein.

Mit der Dialysemaschine nach dem ersten Aspekt der Erfindung können in vorteilhafter Weise zahlreiche verschiedene Therapieformen, wie zum Beispiel Hämodialyse (HD), Hämofiltration (HF) oder Hämodiafiltration (HDF), mit einer Externfluidik durchgeführt werden. Die Externfluidik kann dabei für alle Anwendungen gleich ausgebildet sein, was besonders anwenderfreundlich ist. Des Weiteren können zusätzliche Verfahrensroutinen, wie zum Beispiel Priming (Spülen des Dialysator zu Therapiebeginn), Freispülen des Dialysators während einer Therapie, Befüllen der Externfluidik mit Patientenblut, Substitution während einer HD, HF oder HDF, Bolusgabe während einer Therapie, Messen von Rezirkulation, Reinfundieren von Patientenblut nach einer Therapie oder Aufbereitung des Dialysators für eine Wiederverwendung besonders einfach durchgeführt werden. Es kann zwischen den unterschiedlichen, im Weiteren noch näher erläuterten Substitutionsformen Pre- und Postdilution sowie Mixed-Dilution gewechselt werden, ohne die Behandlung zu unterbrechen. Schließlich kann die Externfluidik nach einer Behandlung einfach entleert werden, was Entsorgungskosten verringert. Es ist nicht mehr erforderlich, für jede Therapie das geeignete Schlauchsystem für die Externfluidik bereit zu halten, was Kosten und Aufwand senkt. Ein Wechsel zwischen Therapieformen ist ohne Wechsel und Rekonnektion der Externfluidik möglich, was das Risiko von minderer Sterilität und Dichtigkeit minimiert. Das Handling beim Vorbereiten einer Dialyse wird vereinfacht, Fehlerquellen werden reduziert, Kosten für zusätzliche Schlauchsysteme oder Kochsalzlösungen zum Spülen des Dialysators entfallen.

Die Aufgabe wird gemäß einem weiteren ggf. unabhängig zu beanspruchenden Aspekt der vorliegenden Erfindung gelöst durch eine Dialysemaschine mit einer Internfluidik gemäß der eingangs gegebenen Definition, wobei die Internfluidik folgende Teile/Elemente hat:
- eine Zuleitung, die dafür angepasst ist, Flüssigkeit (Dialysierflüssigkeit) aus der Internfluidik einem extrakorporalen Blutleitungssystem/Schlauchsystem (bildet eine Externfluidik) mit patientenseitigen Anschlüssen zuzuleiten, um beispielsweise geplante hohe Flüssigkeitsverluste auf der Patientenseite zu kompensieren und
- mindestens eine Kupplungseinrichtung, die strömungstechnisch mit der Zuleitung verbunden sowie insbesondere an deren stromabwärtigen Ende angeordnet oder vorgesehen ist, um eine Schlauchleitung des extrakorporalen Schlauchsystems mit der Zuleitung zu koppeln.

Die Kupplungseinrichtung ist nutzerseitig zugänglich an einer Maschinenfront der extrakorporalen Blutbehandlungsmaschine/Dialysemaschine angeordnet, wobei die Dialysemaschine eine Abflussleitung aufweist, um während der maschinenvor- odernachbereitenden Vorgänge, wie Priming, Freispülen des Dialysators oder Abschließen eines Patienten, Flüssigkeit aus dem extrakorporalen Schlauchsystem abzuleiten, insbesondere mittels der Abflussleitung einem Abfallreservoir zuzuleiten, wobei die Dialysemaschine eine Abdeckung/Klappe aufweist, mittels der die Kupplungseinrichtung abzudecken ist, wenn keine Schlauchleitung des extrakorporalen Schlauchsystems (Blutleitungssystems) mit der Kupplungseinrichtung gekoppelt ist, wobei bei geschlossener Abdeckung/Klappe ein Leitungskurzschluss gebildet wird, oder anders ausgedrückt ein die Kupplungseinrichtung umgebendes abgedichtetes Volumen ausgebildet ist, insbesondere ein gegenüber der Umgebung abgedichtetes Volumen ausgebildet ist, über das die Zuleitung strömungstechnisch mit der Abflussleitung verbunden ist.

Die Zuleitung kann insbesondere eine Dialysierflüssigkeitszuleitung, mit der Dialysierflüssigkeit von dem Dialysierflüssigkeitsreservoir zum Dialysator gefördert werden kann, oder eine Substituatzuleitung, mit der Dialysierflüssigkeit als Substituat dem extrakorporalen Schlauchsystem zugeführt werden kann, sein. Die Abflussleitung kann insbesondere eine Dialysierflüssigkeitsableitung, mit der Dialysierflüssigkeit vom Dialysator zum Abfallreservoir gefördert werden kann, oder eine von der Dialysierflüssigkeitsableitung gesonderte Abflussleitung, mit der Flüssigkeit aus dem extrakorporalen Schlauchsystem dem Abfallreservoir zugeführt werden kann, sein.

Die Abdeckung kann durch einen Nutzer betätigt werden und ist vorzugsweise für diesen einfach zugänglich an der Dialysemaschine, insbesondere an deren Gehäuse, zum Beispiel an der Maschinenfront, angeordnet. Sie kann sich in einem offenen Zustand oder in einem geschlossenen Zustand befinden. Im offenen Zustand kann eine Schlauchleitung der Externfluidik an der Kupplungseinrichtung angeordnet oder mit dieser gekoppelt werden, so dass ein aus der Zuleitung (der Internfluidik) und der Schlauchleitung (der Externfluidik) gebildeter Strömungsweg ausgebildet ist. Im geschlossenen Zustand ist keine Schlauchleitung mit der Kupplungseinrichtung gekoppelt bzw. kann nicht mit dieser gekoppelt sein. Die Abdeckung bildet im geschlossenen Zustand das abgedichtete Volumen oder einen abgedichteten Raum aus, beispielsweise indem sie dichtend am Maschinengehäuse oder der Zuleitung sowie der Abflussleitung anliegt. Innerhalb des mittels der Abdeckung abgedichteten Volumens befinden sich die Kupplungseinrichtung, die Öffnung der Zuleitung sowie die Öffnung der Abflussleitung. Anders ausgedrückt ist das abgedichtete Volumen bzw. der abgedichtete Raum strömungstechnisch mit der Zuleitung sowie der Abflussleitung verbunden. Auf diese Weise wird bei geschlossener Abdeckung ein Strömungsweg ausgebildet, der von der Zuleitung, die in den geschlossenen Raum mündet, über den von der Abdeckung abgedichteten Raum in die Abflussleitung führt. Über diesen Strömungsweg kann die Internfluidik der Dialysemaschine besonders einfach, effizient und insbesondere vollständig gespült, gereinigt und/oder desinfiziert werden. Durch Schließen der Abdeckung wird ein in sich geschlossener Strömungsweg in der Internfluidik gebildet. Diese ist in der genannten Weise vollständig oder je nach Wunsch oder Anforderung auch nur in Teilbereichen zu reinigen und/oder zu desinfizieren.

Unter einer Dialysierflüssigkeit oder einem Substituat im Sinne der vorliegenden Beschreibung ist dabei eine physiologische Flüssigkeit zu verstehen, die mit dem Patienten bzw. dessen Blut in Kontakt gelangt. Es ist daher unbedingt sicherzustellen, dass die in der Dialysemaschine geförderte Flüssigkeit steril und pyrogenfrei ist.

Bei den genannten Pumpen bzw. Fördereinrichtungen der Dialysemaschine kann es sich insbesondere um Zahnradpumpen oder Membranpumpen handeln. Anstelle einer Substituatpumpe kann als Substituatfördereinrichtung beispielsweise ein Proportionalventil, insbesondere mit Durchflussmesser, verwendet werden, mit dem von dem durch die übrigen Pumpen (der Dialysierflüssigkeitspumpe, insbesondere der Dialysierflüssigkeitszuleitungspumpe oder der Dialysierflüssigkeitsableitungspumpe) in der Internfluidik geförderte Flüssigkeit in dem jeweils gewünschten Maß als Substituat abgezweigt wird.

Nach einer Ausführungsform der Erfindung kann die Abflussleitung zur Dialysierflüssigkeitsableitung führen (und über diese zum Abfallreservoir) und strömungstechnisch verbunden sein. Sie kann insbesondere von der Kupplungseinrichtung zur Dialysierflüssigkeitsableitung führen, so dass innerhalb der Internfluidik ein Strömungsweg ausgebildet ist oder werden kann, der von der Kupplungseinrichtung über die Abflussleitung und die Dialysierflüssigkeitsableitung zum Abfallreservoir führt. Die strömungstechnische Verbindung zwischen der Abflussleitung und der Dialysierflüssigkeitsableitung kann mit einem Absperrventil zu unterbrechen sein. Auf diese Weise können in der Dialysemaschine zahlreiche Strömungswege ausgebildet werden, insbesondere solche, die eine strömungstechnische Verbindung zwischen der Externfluidik und dem Abfallreservoir bilden beispielsweise zur Vor- und Nachbereitung der Dialysemaschine. Eine solche Verbindung kann mit besonderem Vorteil ausgebildet oder geschaltet werden, ohne dass mit dem zur Substituatzuleitung zugehörigen Kupplungsstück verbundene oder gekoppelte Leitungen der Externfluidik umgesteckt werden müssten.

Nach einer weiteren Ausführungsform kann die Internfluidik zwei Substituatzuleitungen mit jeweils einer Kupplungseinrichtung zum Anschließen des extrakorporalen Schlauchsystems aufweisen. Auf diese Weise können beide Substituatzuleitungen oder kann eine der Substituatzuleitungen mit der Externfluidik verbunden sein oder werden. Des Weiteren können beide Substituatzuleitungen oder eine der Substituatzuleitungen mit Absperr- oder Flussregelventilen sowie ggf. einer eigenen Substituatpumpe versehen sein, so dass die durch die jeweilige Substituatzuleitung strömende Flüssigkeit individuell einstellbar und/oder regelbar ist. Eine Substituatzuleitung kann für eine Verbindung strömungstechnisch vor (Pre-Dilution) und die andere Substituatzuleitung strömungstechnisch nach (Post-Dilution) dem Dialysator verwendet werden. Auf diese Weise kann die Dialysemaschine sowohl für eine Pre-Dilution als auch für eine Post-Dilution betrieben werden. Ein Mischbetrieb ist außerdem möglich, indem über beide Substituatzuleitungen gleichzeitig gefördert wird (Mixed-Dilution). Ein Wechsel zwischen den genannten Betriebsmodi sowie deren Einstellung während einer Therapie ist jederzeit möglich. Es ist besonders bevorzugt, dass eine Substituatleitung, im Folgenden als Substituathauptleitung bezeichnet, von der Dialysierflüssigkeitszuleitung abzweigt, wobei die Substituatpumpe in dieser Substituathauptleitung angeordnet ist, die sich stromab der Substituatpumpe in die vorgenannten zwei Substituatzuleitungen aufteilt. Dabei kann der Substituatfluss mittels nur einer Pumpe bewirkt werden, wobei die Aufteilung des Substituatflusses auf die der Pumpe nachfolgenden Substituatzuleitungen mittels der genannten Flussregelventile erfolgt.

Nach einer Ausführungsform kann die Internfluidik zwei Abflussleitungen mit jeweils einer Kupplungseinrichtung zum Anschließen des extrakorporalen Schlauchsystems aufweisen. Beide Abflussleitungen können über ihre jeweilige Kupplungseinrichtung mit der Externfluidik verbunden sein oder werden. Des Weiteren können beide Abflussleitungen oder eine der Abflussleitungen mit Absperr- oder Flussregelventilen versehen sein, so dass die durch die jeweilige Abflussleitung strömende Flüssigkeit individuell einstellbar und/oder regelbar ist.

Es ist besonders vorteilhaft, wenn die zwei Abflussleitungen strömungstechnisch miteinander verbunden sind. Dabei kann zwischen den Abflussleitungen und/oder in einer oder in jeder Abflussleitung ein Absperrventil oder Regelventil angeordnet sein. Bei dieser Ausführungsform ist vorteilhaft, dass der Fluss durch jede Abflussleitung (und damit der Fluss durch die jeweils zugehörige Kupplung und die daran angeschlossene Leitung der Externfluidik) mittels des Absperr- oder Regelventils eingestellt werden kann, ohne dass eine Änderung der Konnektion von Externfluidik und Interfluidik notwendig ist.

Bei einer Ausführungsform kann die Substituatzuleitung bzw. eine der oder beide Substituatzuleitungen strömungstechnisch mit der Abflussleitung verbunden sein. Diese Verbindung ist ausschließlich innerhalb der Internfluidik realisiert und kann insbesondere über eine gesonderte Leitung (auch als Verbindungsleitung bezeichnet) erfolgen, die von der Substituatzuleitung zur Abflussleitung führt. Zwischen der Substituatzuleitung und der Abflussleitung kann ein Absperrventil oder Regelventil angeordnet sein. Es ist bei dieser Ausführungsform von besonderem Vorteil, dass über die Verbindungsleitung eine Verbindung von der Substituatzuleitung zur Abflussleitung und damit zum Abfallreservoir bewirkt werden kann, welche Verbindung ausschließlich innerhalb der Internfluidik ausgebildet ist. Es besteht so zum Beispiel die Möglichkeit, die Substituatleitungen zu reinigen oder zu spülen, ohne dass dazu eine Externfluidik angeschlossen sein oder für den Spülvorgang verwendet werden muss. Daher kann auf die Verwendung eines ansonsten üblichen Einwegprodukts verzichtet werden.

Optional kann darüber hinaus in der Abflussleitung bzw. in einer oder in beiden Abflussleitungen ein Luftabscheider angeordnet sein.

Bei einer Ausführungsform der Erfindung kann die Abdeckung, durch die die Kupplungseinrichtung abzudecken ist, im geschlossenen Zustand dichtend an der Maschinenfront anliegen. Auf diese Weise wird besonders einfach ein die Kupplungseinrichtung umgebendes Strömungsvolumen ausgebildet, über das diese strömungstechnisch mit der Abflussleitung verbunden ist bzw. zu verbinden ist. Die Abdeckung kann insbesondere im geschlossenen Zustand und/oder im offenen Zustand verriegelbar sein, so dass sie in dem jeweiligen Zustand sicher verbleibt und Dichtigkeit des mittels der geschlossenen Abdeckung ausgebildeten Strömungswegs sichergestellt ist. Sie kann des Weiteren insbesondere in den geschlossenen Zustand vorgespannt sein, insbesondere mittels einer Feder oder eines ähnlichen Spannelements, so dass ein versehentliches Austreten von Flüssigkeit verhindert werden kann.

Bei einer Ausführungsform kann die Substituatpumpe stromauf zu einer Verzweigung zu den Kupplungseinrichtungen angeordnet sein und stromab zu der Verzweigung in jeder Substituatzuleitung ein Steuerungsventil zur wahlweisen Steuerung einer Pre- und/oder Post-Dilution eingesetzt sein.

Bei einer Ausführungsform kann in der Substituatzuleitung ein Drucksensor, insbesondere ein Influss-Drucksensor angeordnet sein. Hierrunter ist ein Drucksensor zu verstehen, der in direktem Fluidkontakt steht und so den Druck direkt im Fluid/in der Flüssigkeit misst. Vorzugsweise kann sich zwischen dem Fluid und dem Drucksensor noch ein Luftvolumen befinden, in welchem im statischen Fall der gleiche Druck wie im Fluid herrscht. Da jedoch Luft kompressibel ist, kann dadurch ein oszillierendes Drucksignal, wie es auf der Blutseite vorliegt, gedämpft werden. Über diesen Drucksensor kann der Druck in der Substituatzuleitung und damit auch der Druck in Blutschläuchen der Externfluidik erfasst werden. Durch solche Drucksensoren bzw. eine solche Anordnung der Drucksensoren kann die Messgenauigkeit des Drucks in der Externfluidik im Vergleich zu herkömmlichen Sensoren, bei denen über eine Luftsäule gemessen wird, verbessert werden, da die Dämpfung des Signals durch die Luftsäule entfällt. Somit kann zum Beispiel eine venöse Nadeldiskonnektion (VND) besser als beim Stand der Technik erkannt werden.

Es sei angemerkt, dass die erfindungsgemäße Dialysemaschine Bolusgaben grundsätzlich ermöglicht. Bei einer Bolusgabe einer physiologischen Flüssigkeit oder physiologischen Lösung, also einem Infundieren dieser Flüssigkeit in den Blutkreislauf eines Patienten, ist eine kurzzeitige Abschaltung des Bilanzsystems erforderlich. Infolgedessen können die Zuleitungspumpe und die Ableitungspumpe mit unterschiedlichen Förderraten betrieben werden. Die Differenz dieser Förderraten entspricht dann der Bolusgabe in den Patienten. Es sei angemerkt, dass eine Bolusgabe genutzt werden kann, um die Rezirkulation zu bestimmen. Aufgrund der Bolusgabe wird eine Verdünnung des Hämatokrits sowie eine Verringerung von Konzentrationen urämischer Substanzen bewirkt. Im Falle einer Rezirkulation kommt es in der arteriellen Zuleitung der Externfluidik zu einer Änderung des Hämatokrits oder zu einer Konzentration urämischer Substanzen, was bei einer Überwachung der Dialyseeffektivität, insbesondere bei einem Echtzeitmonitoringverfahren, zu einer Signaländerung führt, so dass auf diese Weise auf eine Rezirkulation geschlossen werden kann.

Die Erfindung wird im Folgenden anhand beispielhafter, nicht einschränkender und in den anhängenden Figuren gezeigter Ausführungsformen näher erläutert. Dabei zeigt:
Figur 1 eine schematische Darstellung einer ersten Ausführungsform einer Dialysemaschine nach der Erfindung,
Figur 2 eine schematische Darstellung einer zweiten Ausführungsform einer Dialysemaschine nach der Erfindung,
Figur 3 eine schematische Darstellung des Kupplungsbereichs zwischen Externfluidik und Internfluidik in einer Ausgestaltung,
Figur 4 eine schematische Darstellung des Kupplungsbereichs zwischen Externfluidik und Internfluidik in einer anderen Ausgestaltung als Figur 3,
Figur 5 eine Schnittdarstellung einer Kupplung zwischen Internfluidik und Externfluidik,
Figur 6 eine schematische Darstellung von Flusswegen nach dem Stand der Technik für eine Post-Dilution,
Figur 7 eine schematische Darstellung von Flusswegen bei einer Dialysemaschine nach einem nicht beanspruchten Aspekt der Erfindung während einer Therapie,
Figur 8 eine schematische Darstellung von Flusswegen bei einer Dialysemaschine nach der Erfindung beim Spülen des Dialysators,
Figur 9 eine schematische Darstellung alternativer Flusswege bei einer Dialysemaschine nach der Erfindung beim Spülen des Dialysators,
Figur 10 eine schematische Darstellung von Flusswegen bei einer Dialysemaschine nach der Erfindung zur Konnektion eines Patienten,
Figur 11 eine schematische Darstellung von Flusswegen bei einer Dialysemaschine nach der Erfindung zur Reinfundierung von Blut in einen Patienten,
Figur 12 eine schematische Darstellung alternativer Flusswege bei einer Dialysemaschine nach der Erfindung zur Reinfundierung von Blut in einen Patienten,
Figur 13 eine schematische Darstellung von Flusswegen bei einer Dialysemaschine nach der Erfindung zur Entleerung der Externfluidik nach einer Behandlung,
Figur 14 eine schematische Darstellung von Flusswegen bei einer Dialysemaschine nach der Erfindung zur Aufbereitung des Dialysators für eine Wiederverwendung (Re-Use),
Figur 15 eine schematische Schnittdarstellung einer weiteren erfindungsgemäßen Möglichkeit zur Kopplung einer Externfluidik mit einer Internfluidik und
Figur 16 eine schematische Schnittdarstellung einer weiteren erfindungsgemäßen Kopplung einer Externfluidik mit einer Internfluidik.

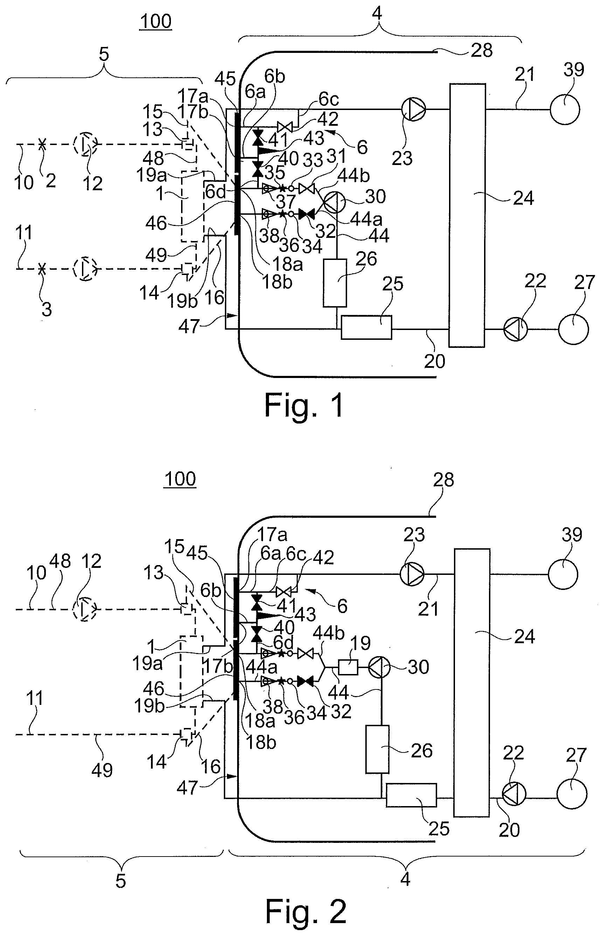

Figur 1 zeigt einen Teil einer ersten Ausführungsform einer Dialysemaschine 100 nach der Erfindung in einer schematischen Darstellung. Diese weist eine Internfluidik 4 und eine Externfluidik 5 auf. Letztere wird im Allgemeinen auch als extrakorporales Blutsystem oder extrakorporaler Blutkreislauf bezeichnet und ist in der Figur gestrichelt dargestellt.

Die Internfluidik 4 ist im Wesentlichen vollständig in einem in der Figur 1 schematisch angedeuteten Maschinengehäuse 28 der Dialysemaschine 100 aufgenommen. Diese weist eine Maschinenseite bzw. Maschinenfront 47 auf, die durch das Maschinengehäuse 28 gebildet sein kann und für einen Nutzer, in der Regel ein Patient oder medizinisches Personal, zur Bedienung und Handhabung der Dialysemaschine 100 zugänglich ist. Die Interfluidik weist ein Dialysierflüssigkeitsreservoir 27 und vorzugsweise ein Abfallreservoir 39 auf. Mittels einer Dialysierflüssigkeitszuleitungspumpe 22, im Folgenden auch als Zuleitungspumpe 22 bezeichnet, wird Dialysierflüssigkeit über eine Dialysierflüssigkeitszuleitung 19b, 20 zu einem in der Regel außerhalb des Maschinengehäuses 28 angeordneten Dialysator 1 gefördert. Von diesem führt eine Dialysierflüssigkeitsableitung 19a, 21 zum Abfallreservoir 39. Dialysierflüssigkeit wird mittels einer Dialysierflüssigkeitsableitungspumpe 23, im Folgenden auch als Ableitungspumpe 23 bezeichnet, durch die Dialysierflüssigkeitsableitung 21 gefördert.

Durch die Dialysierflüssigkeitszuleitung 20 sowie die Dialysierflüssigkeitsableitung 21 geförderte Dialysierflüssigkeit wird mittels eines in der Figur 1 schematisch angedeuteten Bilanzsystems 24 in bekannter Weise erfasst und bilanziert.

Die Internfluidik 4 weist eine Substituatpumpe 30 auf, mit der ein Teil des durch die Dialysierflüssigkeitszuleitung 20 geförderten Dialysierflüssigkeitsstroms maschinenintern abgezweigt und durch eine Substituatzuleitung 44 zur Externfluidik 5 geleitet wird. Die Substituatpumpe 30 ist durch eine in der Figur nicht dargestellte Rechnereinheit, die zum Beispiel zum Bilanzsystem 24 gehören kann, gesteuert. In der Dialysierflüssigkeitszuleitung 20 ist ein Filter 25 und in der Substituatleitung 44 ein Filter 26 angeordnet.

Stromab der Substituatpumpe 30 teilt sich die Substituatzuleitung 44 in zwei Substituatzuleitungen 44a, 44b auf. Die Substituatzuleitung 44a dient in der gezeigten Verschaltung mit der Externfluidik 5 einer Zuleitung von Dialysierflüssigkeit Post-Dilution, während die Substituatzuleitung 44b einer Zuleitung von Dialysierflüssigkeit Pre-Dilution dient. Entsprechend der Verschaltung mit der Externfluidik 5 kann dies umgekehrt werden bzw. sein. In der Substituatzuleitung 44a ist stromab der Pumpe 30 ein Steuerungsventil 32 zur Steuerung der Post-Dilution angeordnet, gefolgt von einem Druckaufnehmer 34, einem Rot-Detektor 36 zur Bluterkennung und einem Rückschlagventil 38. In der Substituatzuleitung 44b ist stromab der Pumpe 30 ein Steuerungsventil 31 zur Steuerung der Pre-Dilution angeordnet, gefolgt von einem Druckaufnehmer 33, einem Rot-Detektor 35 zur Bluterkennung und einem Rückschlagventil 37. Es sei angemerkt, dass anstelle der Ventile 31 und 32 ein Proportionalventil, insbesondere ein einziges Proportionalventil, verwendet werden kann. Durch eine Steuerung der maschineninternen Ventile 31, 32 kann zwischen Pre- und Post-Dilution bzw. Pre- und Post-Bolusgabe gewählt oder gewechselt werden. Es besteht außerdem die Möglichkeit einer Mixed-Dilution, bei der eine Substitution bzw. Bolusgabe sowohl pre als auch post erfolgt. Die Verbindung oder Kopplung zwischen den Substituatzuleitungen 44a, 44b einerseits und der Externfluidik 5 andererseits wird im weiteren Verlauf beschrieben.

Die Internfluidik 4 weist eine Abflussleitung 6 auf. Diese ist gesondert zur Dialysierflüssigkeitsableitung 21 vorgesehen. Im dargestellten Ausführungsbeispiel besteht die Abflussleitung 6 im Wesentlichen aus zwei Abflussleitungen 6a und 6b sowie einem Abflussleitungsabschnitt 6c. Die Abflussleitungen 6a und 6b führen von Anschlüssen oder Kupplungen 17a bzw. 17b, die an der Maschinenfront 47 angeordnet sind und später detaillierter beschrieben werden, zum Abflussleitungsabschnitt 6c, in den sie sich vereinen, und von dort zur Dialysierflüssigkeitsableitung 21. Im Abflussleitungsabschnitt 6c ist ein erstes Abflussventil 42 angeordnet, mit dem die gesamte Abflussleitung 6 gegenüber der Dialysierflüssigkeitsableitung 21 abgesperrt werden kann. In der Abflussleitung 6b ist ein zweites Abflussventil 41 angeordnet, mit dem die Abflussleitung 6b gegenüber der Abflussleitung 6a bzw. dem Abflussleitungsabschnitt 6c abgesperrt werden kann. Stromauf des zweiten Abflussventils 41 ist ein Luftabscheider 43 angeordnet.

Die Abflussleitung 6 ist mittels einer Verbindungsleitung 6d mit der Substituatzuleitung 44b verbunden. Die Verbindungsleitung 6d kann als Teil der Abflussleitung 6 betrachtet werden. In ihr ist ein drittes Abflussventil 40 angeordnet, mit dem die Verbindung zwischen der Substituatzuleitung 44b einerseits und der Abflussleitung 6 und damit zur Dialysierflüssigkeitsableitung 21 zu unterbrechen ist.

Die Externfluidik 5, die zusammen mit der erfindungsgemäßen Dialysemaschine 100 genutzt wird, ist einfach und für zahlreiche unterschiedliche Therapieverfahren gleich. Mit der gezeigten Externfluidik 5 kann die Dialysemaschine 100 beispielsweise zur Hämodialyse (HD), Hämofiltration (HF) oder Hämodiafiltration (HDF) genutzt werden. Des Weiteren können Verfahrensroutinen, wie zum Beispiel Priming (Spülen des Dialysator zu Therapiebeginn), Freispülen des Dialysators während einer Therapie, Befüllen der Externfluidik mit Patientenblut, Substitution während einer HD, HF oder HDF, Bolusgabe während einer Therapie, Messen von Rezirkulation, Reinfundieren von Patientenblut nach einer Therapie oder Aufbereitung des Dialysators 1 für eine Wiederverwendung besonders einfach und insbesondere ohne Wechsel der Externfluidik 5 durchgeführt werden. Auch kann die Externfluidik 5 nach einer Behandlung einfach entleert werden. Sie besteht im Wesentlichen aus einem arteriellen Blutschlauch 48, einem venösen Blutschlauch 49, einem Pre-Zuleitungsblutschlauch 15 und einem Post-Zuleitungsblutschlauch 16. Der arterielle Blutschlauch 48 führt von einem arteriellen Anschluss 10, über den er mit einem Patienten verbunden ist, über eine Blutpumpe 12 zum Dialysator 1. Der venöse Blutschlauch 49 führt vom Dialysator 1 zu einem venösen Anschluss 11, über den er mit dem Patienten verbunden ist. Der arterielle Blutschlauch 48 ist mittels einer arteriellen Klemme 2 abzuklemmen. Der venöse Blutschlauch 49 ist mittels einer venösen Klemme 3 abzuklemmen.

Der arterielle Blutschlauch 48 weist zwischen der Blutpumpe 12 und dem Dialysator 1 einen Pre-Konnektor 13 auf. Über diesen ist der Pre-Zuleitungsblutschlauch 15 mit dem arteriellen Blutschlauch 48 und damit auch dem Dialysator 1 strömungstechnisch verbunden. Der venöse Blutschlauch 49 weist stromab des Dialysators 1 einen Post-Konnektor 14 auf. Über diesen ist der Post-Zuleitungsblutschlauch 16 mit dem venösen Blutschlauch 49 und damit auch dem Dialysator 1 strömungstechnisch verbunden. Die Konnektoren 13 und/oder 14 können in Form eines Luftblasenfängers (Entlüftungseinrichtung bekannter Bauart), zum Beispiel an einem Druckaufnehmer, der Externfluidik ausgebildet sein, wie die üblicherweise bei solchen Schlauchsystemen vorhanden sind. Eine Konnektion kann auch über ein T-Stück erfolgen, das in den arteriellen bzw. venösen Blutschlauch 48 bzw. 49 eingebracht ist.

Durch die Blutpumpe 12 kann ein Flüssigkeitsstrom, insbesondere ein Blutstrom, vom arteriellen Anschluss 10 über den Dialysator 1 zum venösen Anschluss 11 erzeugt werden. Beim Durchströmen des Dialysators 1 wird das Blut in bekannter Weise getrennt durch eine in den Figuren nicht dargestellte semipermeable Membran an der Dialysierflüssigkeit vorbeigeführt, die dem Dialysator 1 über die Dialysierflüssigkeitszuleitung 20 zugeführt wird und die über die Dialysierflüssigkeitsableitung 21 vom Dialysator 1 abgeführt wird.

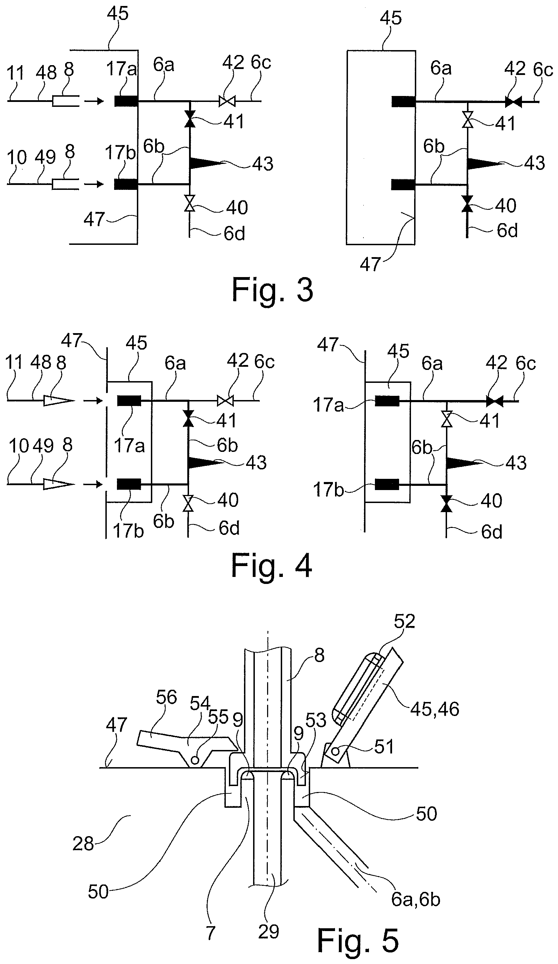

Die Kopplung oder Verbindung zwischen der Internfluidik und der Externfluidik erfolgt über Kupplungseinrichtungen oder Kupplungen 17a, 17b, 18a, 18b. Diese sind für einen Nutzer zugänglich und betätigbar an der Maschinenfront 47 angeordnet und in verschiedenen Ausführungsformen in Figur 3, Figur 4, Figur 5, Figur 15 und Figur 16 detailliert dargestellt.

Die Kupplungseinrichtungen oder Kupplungen 17a, 17b, 18a, 18b können in der Ausführungsform der Figur 3 in Form von Luer-Lock, "Small-Bore-Connector" oder Ähnlichem ausgebildet sein. Die Kupplungen 17a, 17b, 18a, 18b der Figur 3 sind auf der Maschinenfront 47 angeordnet und gegenüber dieser erhaben. Bei der Ausführungsform der Figur 4 können die Kupplungseinrichtungen oder Kupplungen 17a, 17b, 18a, 18b in Form von Dornenkonnektoren ausgebildet sein. Bei dieser Ausführungsform ist in der Maschinenfront 47 im Bereich der Kupplungen 17a, 17b, 18a, 18b eine Vertiefung oder Ausnehmung 66 ausgebildet, in der die Kupplungen 17a, 17b, 18a, 18b angeordnet sind.

Es sind eine Spülklappe 45 (für die Kupplungen 17a und 17b der Abflussleitung 6) bzw. eine Spülklappe 46 (für die Kupplungen 18a, und 18b der Substituatleitungen 44a, 44b) vorgesehen, z.B. an der Maschinenfront 47. Die Spülklappen 45, 46 bilden jeweils eine Abdeckung 45 bzw. 46 für die Kupplungen 17a, 17b bzw. die Kupplungen 18a, 18b aus. Sie können jeweils in eine geöffnete Position/einen geöffneten Zustand und in eine geschlossene Position/einen geschlossenen Zustand gebracht werden. In der geöffneten Position ist eine Zugänglichkeit für die Kupplungen 17a, 17b, 18a, 18b zum Anschließen der Externfluidik 5, insbesondere des arteriellen Blutschlauchs 48, des venösen Blutschlauchs 49, des Pre-Zuleitungsblutschlauchs 15 sowie des Post-Zuleitungsblutschlauchs 16, je nach Art der durchzuführenden Therapie, gegeben. Die Spülklappe 45 ist in der linken Abbildung der Figur 3 bzw. der Figur 4 im geöffneten und in der rechten Abbildung der Figur 3 bzw. der Figur 4 im geschlossenen Zustand gezeigt.

Im geschlossenen Zustand bilden die Spülklappen 45, 46 jeweils ein abgedichtetes Volumen um die jeweiligen Kupplungen 17a, 17b, 18a, 18b aus, beispielsweise indem sie dichtend an der Maschinenfront 47 oder an den Kupplungen 17a, 17b, 18a, 18b selbst anliegen. Bei geschlossener Spülklappe 45 sind die Kupplungen 17a und 17b in dem mittels der Spülklappe 45 abgedichteten Volumen aufgenommen. Bei geschlossener Spülklappe 46 sind die Kupplungen 18a und 18b in dem mittels der Spülklappe 46 abgedichteten Volumen aufgenommen. Anders ausgedrückt wird bei geschlossener Spülklappe 45 ein nach außen abgedichteter Strömungsweg (Kurzschluss) ausgebildet, der von der Abflussleitung 6b über die Kupplung 17b in das durch die geschlossene Spülklappe 45 abgedichtete Volumen und von diesem über die Kupplung 17a zur Abflussleitung 6a führt. In gleicher Weise wird bei geschlossener Spülklappe 46 ein nach außen abgedichteter Strömungsweg (Kurzschluss) ausgebildet, der von der Substituatzuleitung 44a über die Kupplung 18b in das durch die geschlossene Spülklappe 46 abgedichtete Volumen und von diesem über die Kupplung 18a zur Substituatzuleitung 44b führt.

Die in der Figur 2 gezeigte Ausführungsform der Dialysemaschine 100 unterscheidet sich von der in der Figur 1 gezeigten Ausführungsform durch ein weiteres Filter 19, das stromab der Substituatpumpe 30 in der Substituatleitung 44 angeordnet ist. Das Filter 19 dient einem Patientenschutz vor Partikeln. Es kann, muss aber nicht, identisch zu den Filtern 25, 26 ausgebildet sein und zusätzlich oder alternativ zu diesen oder einem von diesen vorgesehen sein. Im Übrigen wird auf die Beschreibung der Ausführungsform der Figur 1 verwiesen.

Eine Ausführungsform einer Kupplung 17a, 17b, 18a, 18b, die alternativ oder zusätzlich zu den zuvor beschriebenen Ausführungsformen genutzt werden kann, ist in Figur 5 detailliert in einer schematischen Schnittdarstellung gezeigt. Zu erkennen ist das Maschinengehäuse 28 mit der Maschinenfront 47. In dieses ist zumindest ein Endabschnitt einer Zuleitung 29 eingebracht. Bei der in der Figur 5 gezeigten Zuleitung 29 kann es sich um eine der Substituatleitungen 44a, 44b oder um eine der Abflussleitungen 6a, 6b handeln. Das maschinenfrontseitige Ende der Zuleitung 29 ist zu einem Kupplungsstück 7 als Kupplung oder Kupplungseinrichtung 17a, 17b, 18a, 18b ausgebildet, an das eine der Schlauchleitungen 15, 16, 48, 49 der Externfluidik 5 mittels eines passenden Konnektors 8 gekoppelt werden kann. Zwischen dem Kupplungsstück 7 und dem Konnektor 8 ist eine Dichtung 9 angeordnet. Das Kupplungsstück 7 ist von einem ringförmigen Kanal 50 umgeben. In diesen mündet eine Abflussleitung 6, 6a, 6b. An der Maschinenfront 47 ist neben dem ringförmigen Kanal 50 eine um ein Scharnier 51 schwenkbar Abdeckung 45, 46 angeordnet. Diese weist an einem Außenrand eine O-Ringdichtung 52 auf, die bei geschlossener Abdeckung 45, 46 gegen eine äußere Wand 53 des ringförmigen Kanals 50 abdichtet. In der Figur 5 ist die Abdeckung 45, 46 in einer geöffneten Position gezeigt. Die Externfluidik 5 ist an die Zuleitung 29 angeschlossen, indem der an eine Schlauchleitung 15, 16, 48, 49 der Externfluidik 5 angeschlossene Konnektor 8 auf das Kupplungsstück 7 aufgeschoben und mittels der Dichtung 9 abgedichtet ist. Der Konnektor 8 ist am Kupplungsstück 7 durch die Wirkung einer Raste 54 gehalten. Diese ist um eine Achse 55 schwenkbar und in die dargestellte Position durch eine Federbelastung vorgespannt, so dass der Konnektor 8 mittels der Raste 54 auf das Kupplungsstück 7 und gegen die Dichtung 9 gedrückt wird. Zum Lösen des Konnektors 8 wird die Raste 54 über einen Hebel 56 betätigt und dadurch um die Achse 55 geschwenkt. Infolgedessen gibt die Raste 54 den Konnektor 8 frei, der dann von dem Kupplungsstück 7 abgezogen werden kann.

Zum Ausbilden eines Kurzschlussströmungswegs von der Zuleitung 29 in die Abflussleitung 6, 6a, 6b wird die Verschlussklappe oder Abdeckung 45, 46 um ihr Scharnier 51 in Richtung der Maschinenfront geschwenkt, bis die Dichtung 52 in den ringförmigen Kanal 50 eingreift und dichtend an dessen Wand 53 anliegt. In dieser geschlossenen Stellung ist die Abdeckung 45, 46 in gleicher Weise wie zuvor bezüglich des Konnektors 8 beschrieben mittels der Raste 54 gehalten und gesichert und kann nur durch eine Betätigung der Raste 54 freigegeben und geöffnet werden. Durch die geschlossene und gegenüber dem Maschinengehäuse 28 abgedichtete Abdeckung 45, 46 wird ein die Kupplungseinrichtung 7 umgebendes abgedichtetes Volumen ausgebildet, in das die Zuleitung 29 mündet und von dem die Abflussleitung 6, 6a, 6b abführt. Eine Reinigung und Desinfektion der Zuleitung zusammen mit der Kupplung ist somit in einfacher und effektiver Weise möglich.

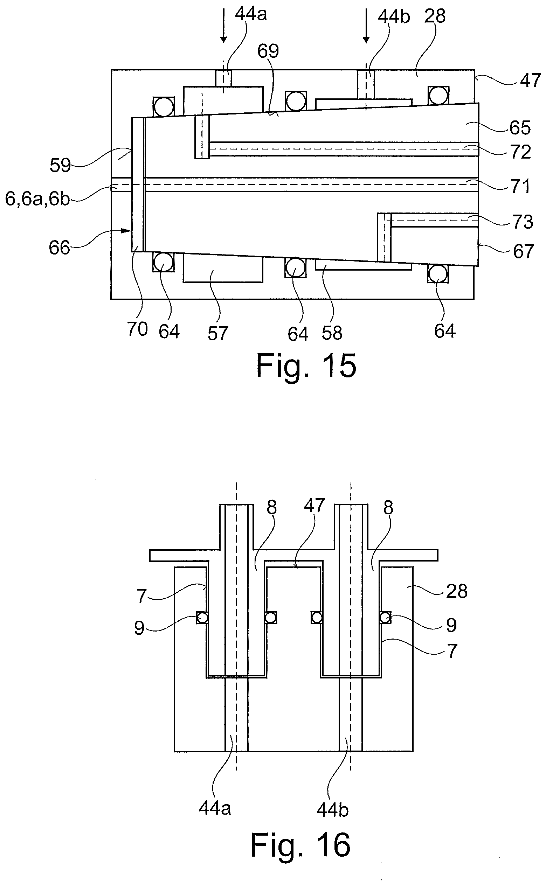

Eine weitere Ausführungsform der Kopplung zwischen der Externfluidik 5 und der Internfluidik 4 ist in Figur 15 gezeigt. Bei dieser Ausführungsform ist in das Maschinengehäuse 28 eine im Wesentlichen kegelstumpfförmige Vertiefung oder Ausnehmung 66 eingebracht, in die eine erste Substituatzuleitung 44a, eine zweite Substituatzuleitung 44b und eine Abflussleitung 6, 6a, 6b münden. In die Vertiefung sind ein erster Ringkanal 57 und ein zweiter Ringkanal 58 eingebracht. Die Ringkanäle 57, 58 sind in der Mantelfläche 69 der Ausnehmung 66 ausgebildet und laufen darin vollumfänglich um. In den ersten Ringkanal 57 mündet die Substituatzuleitung 44a. In den zweiten Ringkanal 58 mündet die Substituatzuleitung 44b. Die Abflussleitung 6, 6a, 6b mündet in die Stirnfläche 59 der Vertiefung 66. Zwischen der Stirnfläche 59, dem ersten Ringkanal 57 sowie dem zweiten Ringkanal 58 ist jeweils ein O-Dichtring 64 angeordnet. An der Maschinenfront 47 kann, obwohl das in Figur 15 nicht gezeigt ist, eine Abdeckung 45, 46 in der zuvor wie mit Bezug auf Figur 5 beschriebenen Weise angeordnet sein.

In die Vertiefung 66 des Maschinengehäuses 28 ist ein als Steckkonnektor ausgebildeter Konnektionsadapter 65 eingebracht. Dieser ist insbesondere für eine Verwendung zusammen mit einer Dialysemaschine nach der Erfindung eingerichtet und ausgebildet. Er besteht vorzugsweise aus Kunststoff und kann insbesondere mittels Spritzgießen hergestellt sein. Er besitzt eine der Mantelfläche 69 der Ausnehmung 66 entsprechende Außenkontur, insbesondere eine kegelstumpfförmige Außenkontur. Der Konnektor 65 ist mittels der O-Dichtringe 64 gegenüber der Mantelfläche 69 der Ausnehmung 66 abgedichtet. Der Durchmesser des Konnektors 65 kann derart ausgebildet sein, dass dieser nicht bis zum Anschlag an die Stirnfläche 59 in die Ausnehmung 66 eingesetzt werden kann, sondern dass zwischen der Stirnfläche 59 und dem Konnektor 65 eine Kavität 70 verbleibt.

Der Konnektor 65 ist mit durchgehenden Kanälen 71, 72, 73 versehen. Der Kanal 71 führt von einer Vorderseite 67 des Konnektors 65 zu dessen Stirnseite 68, die der Stirnfläche 59 der Ausnehmung 66 gegenüber liegt. Die Kanäle 72 und 73 sind mittels sich schneidender Bohrungen ausgebildet und führen von der Vorderseite 67 des Konnektors 65 zu dessen Mantelfläche, und zwar jeweils im Bereich der ringförmigen Kanäle 57, 58. Die Öffnungen der Kanäle 71, 72 und 73 auf der Vorderseite 67 des Konnektors 65 sind in der jeweils gewünschten Weise strömungstechnisch mit einer Leitung oder einem Schlauch des extrakorporalen Blutsystems (der Externfluidik 5) verbunden; dies ist in Figur 15 nicht dargestellt. Durch Einstecken des Konnektors 65 in die Ausnehmung 66 wird eine strömungstechnische Verbindung zwischen der Internfluidik 4 und der Externfluidik 5 bewirkt, wobei mittels einer einzigen Steckverbindung ein erster Strömungsweg (von der Abflussleitung 6 über die Kavität 70 und den Kanal 71), ein zweiter Strömungsweg (von der Dialysierflüssigkeitszuleitung 44a über den ringförmigen Kanal 57 und den Kanal 72) und ein dritter Strömungsweg (von der Dialysierflüssigkeitszuleitung 44b über den ringförmigen Kanal 58 und den Kanal 73) ausgebildet werden. Mittels der O-Dichtringe 64 sind diese drei Strömungswege gegeneinander abgedichtet. Durch Entfernen des Konnektionsadapters 65 und schließen der in Figur 15 nicht dargestellten Klappe 45, 46, die die Ausnehmung oder Vertiefung 66 dichtend gegenüber der Umgebung verschließt, können die beiden Zuleitungen 44a, 44b sowie die Abflussleitung 6 in der zuvor beschriebenen Weise gereinigt und desinfiziert werden.

Eine weitere Kopplung nach der Erfindung ist in Figur 16 gezeigt. Diese entspricht im Wesentlichen der Kopplung nach Figur 4, erfolgt allerdings nicht mittels einzelner Konnektoren 8, sondern mittels eines Doppelkonnektors. Dieser weist zwei Konnektoren 8 auf, die mittels einer Platte miteinander verbunden sind. Eine Konnektion der Externfluidik 5 mit der Internfluidik 4 erfolgt, indem die Konnektoren 8 in Kupplungen 7 eingesteckt werden. Eine Abdichtung erfolgt über O-Dichtringe 9.

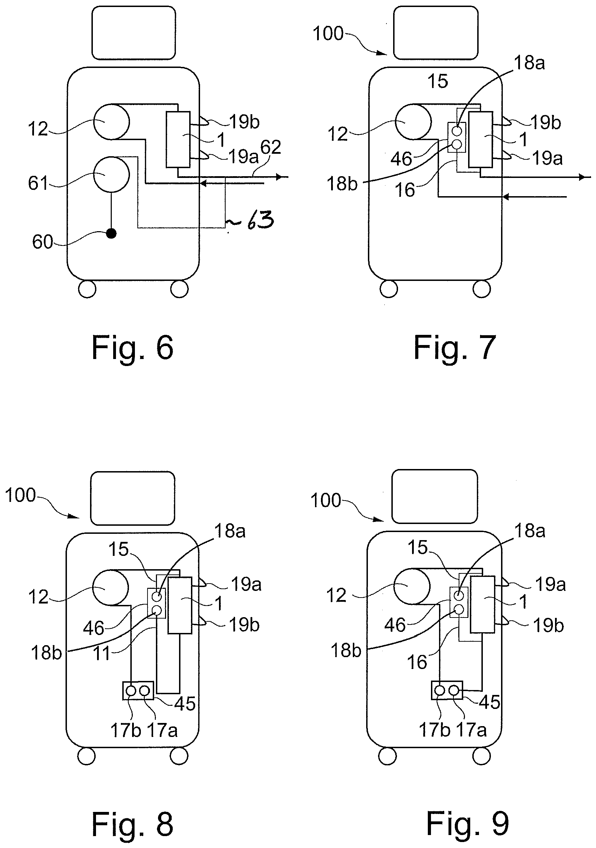

Durch die Erfindung kann die Externfluidik 5 in unterschiedlicher Weise an die Dialysemaschine 100, wie sie in den Figuren 1 und 2 dargestellt ist, angeschlossen werden. Beispielhafte Anschlussmöglichkeiten sind in den Figuren 6 bis 14 schematisch dargestellt und werden im Folgenden erläutert. In den Figuren 6 bis 14 sind aus Gründen einer besseren Übersichtlichkeit nicht alle Bestandteile der Dialysemaschine 100 gezeigt und/oder gekennzeichnet.

Figur 6 zeigt den Flussweg für eine HDF mit Post-Dilution bei einer herkömmlichen Dialysemaschine nach dem Stand der Technik. Neben der Blutpumpe 12 ist an der Maschinenfront 47 eine gesonderte, ebenfalls für einen Nutzer zugängliche Substituatpumpe 61 angeordnet. Substituat wird von einem gesonderten Substituatzugang 60 mittels der Substituatpumpe 61 durch einen Substituatschlauch 63 einer Konnektion 62 zugeführt und dort in den extrakorporalen Blutkreislauf 5 eingebracht. Der Substituatschlauch 63 gehört zur Externfluidik 5 und muss je nach Art der anzuwendenden Therapie in unterschiedlichen Ausführungsformen und Konnektionen vorgesehen sein und vorliegen.

Figur 7 zeigt den Flussweg bei einer Dialysemaschine 100 nach einem nicht beanspruchten Aspekt der Erfindung während einer Therapie, wobei die Externfluidik 5 mittels des arteriellen Anschlusses 10 und des venösen Anschlusses 11 an den Blutkreislauf eines in der Figur nicht gezeigten Patienten angeschlossen ist. Im Gegensatz zur Figur 6 sind die Substituatpumpe 61, der Substituatschlauch 63 und die Konnektion 62 entfallen. Von der Externfluidik 5 ist gezeigt der arterielle Blutschlauch 48 mit dem arteriellen Anschluss 10, der venöse Blutschlauch 49 mit dem venösen Anschluss 11, der Pre-Zuleitungsblutschlauch 15 und der Post-Zuleitungsblutschlauch 16 sowie die Blutpumpe 12. Die dargestellten Verbindungen ermöglichen eine Pre- und eine Post-Dilution und ersetzen die bisher erforderlichen Verbindungen zu den einzelnen Drucksensoren.

Figur 8 zeigt den Flussweg bei einer Dialysemaschine 100 nach der Erfindung bei einem Spülen des Dialysators 1 bzw. für ein Befüllen der Externfluidik 5 mit physiologischer Flüssigkeit. Dabei wird über die Substituatleitung 44a und die Kupplung 18a Spülflüssigkeit (physiologische Lösung) in die Post-Zuleitung 16 vor dem venösen Anschluss 11 und damit in den venösen Teil der Externfluidik 5 eingebracht. Die Spülflüssigkeit durchströmt den Dialysator 1 von unten nach oben (anders ausgedrückt vom venösen Teil in den arteriellen Teil), also entgegen der üblichen Strömungsrichtung bei einer Behandlung. Diese Durchströmung mit Spülflüssigkeit wird durch die rückwärts laufende Blutpumpe 12 bewirkt. Nach Durchströmen der Blutpumpe 12 wird die Spülflüssigkeit über den arteriellen Blutschlauch 48, dessen arterieller Anschluss 10 mit der Kupplung 17b gekoppelt ist, der Abflussleitung 6 zugeführt und über diese in das Abfallreservoir 39 geleitet. Bei einem derartigen Spülvorgang wird Luft mittels des Luftabscheiders 43 aus der Externfluidik 5 entfernt.

Figur 9 zeigt einen alternativen Flussweg bei einer Dialysemaschine 100 nach der Erfindung bei einem Spülen des Dialysators 1 bzw. für ein Befüllen der Externfluidik 5 mit physiologischer Flüssigkeit. Der venöse Blutschlauch 49 ist mit seinem venösen Anschluss 11 an die Kupplung 17a angeschlossen. Der arterielle Blutschlauch 48 ist mit seinem arteriellen Anschluss 10 an die Kupplung 17b angeschlossen. Bei nicht fördernder Blutpumpe 12 und geöffneter venöser Klemme 3 wird der venöse Blutschlauch 49 über den Konnektor 14 (siehe Fig. 1, 2) in Richtung des venösen Anschlusses 11 mit Spülflüssigkeit (physiologische Flüssigkeit) gefüllt. Im venösen Blutschlauch 49 befindliche Luft wird dabei in die Abflussleitung 6 gefördert und aus dem System entfernt. Ist der venöse Blutschlauch 49 vollständig gefüllt, wird die venöse Klemme 3 geschlossen. Die Blutpumpe 12 wird entgegen der üblichen Förderrichtung betrieben. Dadurch wird der Dialysator 1 und die übrige Externfluidik 5, insbesondere der arterielle Blutschlauch 48, mit Spülflüssigkeit befüllt. Eine Entlüftung erfolgt ebenfalls über den Luftabscheider 43. Sind beide Schlauchbereiche, also sowohl der arterielle als auch der venöse Blutschlauch entlüftet, erfolgt eine Zirkulation der Spülflüssigkeit über die kurzgeschlossenen Kupplungen 17a und 17b, bis ein Patient an das System angelegt wird.

Es ist bei diesem Flussweg möglich, dass über den Konnektor 14 (siehe Fig. 1, 2) Flüssigkeit mit einem größeren Volumenstrom in das System eingebracht wird als der durch die Blutpumpe 12 im Rückwärtsbetrieb geförderte Volumenstrom. Eine Volumenstromdifferenz, die mittels der Blutpumpe 12 im Rückwärtsbetrieb nicht gefördert werden kann, strömt in diesem Fall über den venösen Blutschlauch 49 zur Kupplung 17a. Auf diese Weise kann eine synchrone Entlüftung und Befüllung des venösen Blutschlauchs 49 und des arteriellen Blutschlauchs 48 erfolgen.

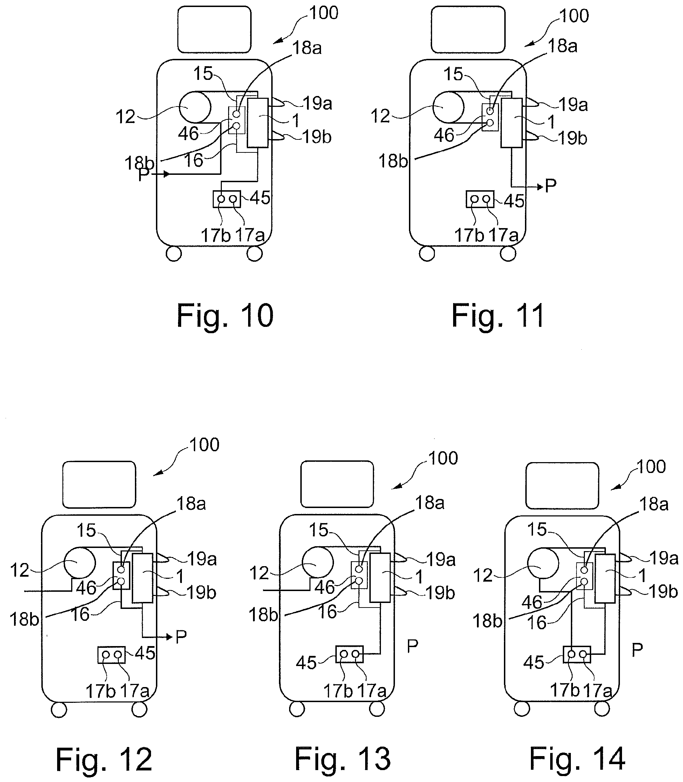

Figur 10 zeigt einen Flussweg bei einer Dialysemaschine 100 nach der Erfindung bei einer Konnektion eines nicht dargestellten Patienten. Dieser Flussweg kann insbesondere nach einem Befüllen und/oder Spülen mittels eines der Flusswege der Figuren 8 und 9 eingerichtet oder durchgeführt werden. Blut des Patienten wird mittels der vorwärts betriebenen Blutpumpe 12 über den arteriellen Anschluss 10 in den arteriellen Blutschlauch 48 der Externfluidik gefördert. Durch Einströmen von Blut in die Externfluidik 5 wird darin befindliche physiologische Flüssigkeit verdrängt und über die Kupplung 17b in die Abflussleitung 6 und von dieser in das Abfallreservoir gefördert. Im Bereich der venösen Klemme 3 kann in dem venösen Blutschlauch 49 ein Rotdetektor angeordnet sein. Dieser wird dabei vorzugsweise genutzt, um den Zeitpunkt zu erfassen, an dem Blut des Patienten an der Klemme 3 ankommt und der venöse Blutschlauch 49 mit dem venösen Anschluss 11 mit dem Blutkreislauf des Patienten verbunden werden soll.

Es sei darauf hingewiesen, dass zum Zwecke eines volumenstabilen Anschließens eines Patienten unmittelbar von dem in Figur 9 dargestellten Flussweg zum Spülen und/oder Befüllen der Externfluidik 5 zu dem in Figur 7 dargestellten Flussweg zur Therapie des Patienten gewechselt werden kann.

Figur 11 zeigt einen Flussweg bei einer Dialysemaschine 100 nach der Erfindung bei einer Reinfundierung von Blut nach einer Behandlung zurück in den Blutkreislauf eines nicht dargestellten Patienten. Dabei wird der arterielle Blutschlauch 48 vom Blutkreislauf des Patienten entkoppelt und auf die Kupplung 18b aufgesteckt. In der Externfluidik 5 befindliche physiologische Flüssigkeit wird dann über die Kupplung 18b in den arteriellen Anschluss 10 in den arteriellen Blutschlauch 48 gefördert. Alternativ oder zusätzlich kann physiologische Flüssigkeit über die Kupplung 18a in die Pre-Zuleitung 15 der Externfluidik 5 und von dieser über den Konnektor 13 in den arteriellen Blutschlauch 48 gefördert werden. Es ist außerdem möglich, physiologische Flüssigkeit über die Kupplung 18b in die Post-Zuleitung 16 der Externfluidik 5 und von dieser über den Konnektor 14 in den venösen Blutschlauch 49 zu fördern. Durch das Einbringen von physiologischer Flüssigkeit in die Externfluidik 5 wird darin befindliches Blut zurück in den Blutkreislauf des Patienten gedrängt.

Figur 12 zeigt einen alternativen Flussweg bei einer Dialysemaschine 100 nach der Erfindung für eine Reinfundierung von Blut nach einer Behandlung zurück in den Blutkreislauf eines nicht dargestellten Patienten. Der arterielle Anschluss 10 wird dabei durch Öffnen der arteriellen Klemme 2 zur Atmosphäre geöffnet. Mittels der Blutpumpe 12 wird daraufhin Luft aus der Atmosphäre in den arteriellen Blutschlauch 48 gefördert. Wenn die Luft den Konnektor 13 erreicht, wird die Förderung der Blutpumpe 12 eingestellt. Über die Kupplung 18a wird daraufhin physiologische Flüssigkeit aus der Internfluidik 4 via der Pre-Zuleitung und den Konnektor 13 in die Externfluidik 5 gefördert, wodurch Blut aus dieser in der zuvor beschriebenen Weise zurück in den Blutkreislauf des Patienten gedrängt wird.

Figur 13 zeigt einen Flussweg bei einer Dialysemaschine 100 nach der Erfindung zur Entleerung der Externfluidik 5 nach einer Reinfundierung von Blut zurück in den Blutkreislauf eines nicht dargestellten Patienten. In der dargestellten Weise ist es möglich, die gesamte Externfluidik 5 vollständig zu entleeren. Dadurch wird deren Gewicht sowie Entsorgungskosten gesenkt. Der arterielle Anschluss 10 ist dabei durch Öffnen der arteriellen Klemme 2 zur Atmosphäre geöffnet. Der venöse Anschluss 11 ist auf die Kupplung 17a aufgesteckt. Über die Dialysierflüssigkeitsableitungspumpe 23 wird in der Externfluidik 5 sowie in der Abflussleitung 6 befindliche physiologische Flüssigkeit in das Abfallreservoir 39 gefördert.

Figur 14 zeigt einen Flussweg bei einer Dialysemaschine 100 nach der Erfindung zur Aufbereitung des Dialysators 1 während einer Desinfektion für eine Wiederverwendung. Der arterielle Anschluss 10 ist dabei auf die Kupplung 17b aufgesteckt. Der venöse Anschluss 11 ist auf die Kupplung 17a aufgesteckt. Bei diesem Flussweg strömt eine Desinfektionsflüssigkeit entweder über die Kupplung 18b über und die den Dialysator 1 und die rückwärtslaufende Blutpumpe zur Kupplung 17b und von dort über die Abflussleitung 6 zum Abfallreservoir 39 oder über die Kupplung 18a über den Dialysator 1 zur Kupplung 17a. Nach der Desinfektion kann über den gleichen Flussweg mit keimfreien Wasser gespült werden, um das Desinfektionsmittel aus dem Dialysator 1 zu entfernen.

| | | | |
|---|---|---|---|
| 1 | Dialysator | 2 | arterielle Klemme |
| 3 | venöse Klemme | 4 | Internfluidik |
| 5 | Externfluidik | 6 | Abflussleitung |
| 6a | Abflussleitung | 6b | Abflussleitung |
| 6c | Abflussleitungsabschnitt | 6d | Verbindungsleitung |
| 7 | Kupplungsstück | 8 | Konnektor |
| 9 | Dichtung | 10 | arterieller Anschluss |
| 11 | venöser Anschluss | 12 | Blutpumpe |
| 13 | Pre-Konnektor | 14 | Post-Konnektor |
| 15 | Pre-Zuleitungsblutschlauch | 16 | Post-Zuleitungsblutschlauch |
| 17a, b | Anschluss/Kupplung | 18a, b | Anschluss/Kupplung |
| 19 | Filter | 19b, 20 | Dialysierflüssigkeitszuleitung |
| 19a, 21 | Dialysierflüssigkeitsableitung | 22 | Dialysierflüssigkeitszuleitungspumpe |
| 23 | Dialysierflüssigkeitsableitungspumpe | 24 | Bilanzsystem |
| 25 | Filter | 26 | Filter |
| 27 | Dialysierflüssigkeitsreservoir | 28 | Maschinengehäuse |
| 29 | Zuleitung | 30 | Substituatpumpe |
| 31 | Steuerungsventil | 32 | Steuerungsventil |
| 33 | Druckaufnehmer | 34 | Druckaufnehmer |
| 35 | Rot-Detektor | 36 | Rot-Detektor |
| 37 | Rückschlagventil | 38 | Rückschlagventil |
| 39 | Abfallreservoir | 40 | Abflussventil |
| 41 | Abflussventil | 42 | Abflussventil |
| 43 | Luftabscheider | 44 | Substituatzuleitung |
| 44a | Substituatzuleitung (post) | 44b | Substituatzuleitung (pre) |
| 45 | Abdeckung/Spülklappe | 46 | Abdeckung/Spülklappe |
| 47 | Maschinenfront | 48 | arterieller Blutschlauch |
| 49 | venöser Blutschlauch | 50 | ringförmiger Kanal |
| 51 | Scharnier | 52 | O-Ringdichtung |
| 53 | äußere Wand | 54 | Raste |
| 55 | Achse | 56 | Hebel |
| 57 | Ringkanal | 58 | Ringkanal |
| 59 | Stirnfläche | 60 | Substituatzugang |
| 61 | Substituatpumpe | 62 | Konnektion |
| 63 | Substituatschlauch | 64 | O-Dichtring |
| 65 | Konnektionsadapter/Konnektor | 66 | Vertiefung/Ausnehmung |
| 67 | Vorderseite | 68 | Stirnseite |
| 69 | Mantelfläche | 70 | Kavität |
| 71 | Kanal | 72 | Kanal |
| 73 | Kanal | 100 | Dialysemaschine |

## Patentansprüche

1. Dialysemaschine (100) mit einer Internfluidik (4),
wobei die Internfluidik (4), folgende Bestandteile aufweist:
- eine Dialysierflüssigkeitspumpe (22, 23), die dafür angepasst ist, Dialysierflüssigkeit von einem Dialysierflüssigkeitsreservoir (27) durch eine Dialysierflüssigkeitszuleitung (20) zu einem Dialysator (1) und von dem Dialysator (1) durch eine Dialysierflüssigkeitsableitung (21) zu einem Abfallreservoir (39) oder zu einer Abfallleitung zu pumpen,
- eine Substituatpumpe (30), die dafür angepasst ist, Dialysierflüssigkeit durch eine Substituatzuleitung (44, 44a, 44b) einem extrakorporalen Blutleitungssystem (5) mit patientenseitigen Anschlüssen (10, 11) zuzuführen, und
- Kupplungseinrichtungen (17a, 17b, 18a, 18b) zum Anschließen des extrakorporalen Blutleitungssystems (5) an die Internfluidik (4),
**dadurch gekennzeichnet, dass** die Internfluidik (4) eine Abflussleitung (6) aufweist, die im Wesentlichen aus zwei Abflussleitungen (6a, 6b) besteht, die von zwei Kupplungseinrichtungen (17a, 17b), die zum Anschließen des extrakorporalen Blutleitungssystems (5) angepasst sind, zu einem Abflussleitungsabschnitt (6c) führen, in dem sie sich vereinen und von dort zu der Dialysierflüssigkeitsableitung (21) und zum Abfallreservoir (39), und zwei Substituatzuleitungen (44a, 44b) mit jeweils einer Kupplungseinrichtung (18a, 18b) zum Anschließen des extrakorporalen Blutleitungssystems (5).

2. Dialysemaschine nach Anspruch 1, **dadurch gekennzeichnet, dass** die zwei Abflussleitungen (6a, 6b) strömungstechnisch miteinander verbunden sind.

3. Dialysemaschine nach einem der vorstehenden Ansprüche, **dadurch gekennzeichnet, dass** die Substituatzuleitung (44, 44a, 44b) strömungstechnisch mit der Abflussleitung (6, 6a, 6b) verbunden ist.

4. Dialysemaschine nach einem der vorstehenden Ansprüche, **dadurch gekennzeichnet, dass** in der Abflussleitung (6, 6a, 6b) ein Luftabscheider (43) angeordnet ist.

5. Dialysemaschine nach einem der vorstehenden Ansprüche, **dadurch gekennzeichnet, dass** die Substituatpumpe (30) stromauf zu einer Verzweigung zu den Kupplungseinrichtungen (17a, 17b, 18a, 18b) angeordnet ist und stromab zu der Verzweigung in jeder Substituatzuleitung (44a, 44b) ein Steuerungsventil (31, 32) zur wahlweisen Steuerung einer Pre- und/oder Post-Dilution eingesetzt ist.

## Claims

1. A dialysis machine (100) comprising internal fluidics (4),
the internal fluidics (4) including the following components:
- a dialysis fluid pump (22, 23) adapted to pump dialysis fluid from a dialysis fluid reservoir (27) through a dialysis fluid feed line (20) to a dialyser (1) and from the dialyser (1) through a dialysis fluid drain (21) to a waste reservoir (39) or to a waste line,
- a substitution fluid pump (30) adapted to supply dialysis fluid through a substitution fluid feed line (44, 44a, 44b) to an extracorporeal blood conduit system (5) having patient-side ports (10, 11), and
- coupling means (17a, 17b, 18a, 18b) for connecting the extracorporeal blood conduit system (5) to the internal fluidics (4),
**characterized in that** the internal fluidics (4) includes a drain line (6), consisting essentially of two drain lines (6a, 6b), leading from two coupling means (17a, 17b), which are adapted for connecting the extracorporeal blood conduit system (5), to a drain line portion (6c) in which they merge and from there they lead to the dialysis fluid drain (21) and to the waste reservoir (39), and
two substitution fluid feed lines (44a, 44b) each having a coupling means (18a, 18b) to connect the extracorporeal blood conduit system (5).

2. The dialysis machine according to claim 1, **characterized in that** the two drain lines (6a, 6b) are fluid-communicated.

3. The dialysis machine according to any one of the preceding claims, **characterized in that** the substitution fluid feed line (44, 44a, 44b) is fluid-communicated with the drain line (6, 6a, 6b).

4. The dialysis machine according to any one of the preceding claims, **characterized in that** an air separator (43) is arranged in the drain line (6, 6a, 6b).

5. The dialysis machine according to any one of the preceding claims, **characterized in that** the substitution fluid pump (30) is arranged upstream of a branching to the coupling means (17a, 17b, 18a, 18b) and downstream of the branching, a control valve (31, 32) for selectively controlling pre-dilution and/or post-dilution is employed in each substitution fluid feed line (44a, 44b).

## Revendications

1. Machine de dialyse (100) dotée d'un système fluidique interne (4),
dans laquelle le système fluidique interne (4) présente des composantes suivantes :
- une pompe de liquide de dialyse (22, 23) qui est adaptée pour pomper du liquide de dialyse d'un réservoir de liquide de dialyse (27) par une conduite d'amenée de liquide de dialyse (20) à un dialyseur (1) et du dialyseur (1) par une conduite de sortie de liquide de dialyse (21) à un réservoir de déchets (39) ou à une conduite de déchets,
- une pompe de liquide de substitution (30) qui est adaptée pour acheminer du liquide de dialyse par une conduite d'amenée de liquide de substitution (44, 44a, 44b) à un système extracorporel de conduite de sang (5) avec des raccords destinés au patient (10, 11), et
- des dispositifs de couplage (17a, 17b, 18a, 18b) pour raccorder le système extracorporel de conduite de sang (5) au système fluidique interne (4),
**caractérisée en ce que** le système fluidique interne (4) présente une conduite d'évacuation (6) qui consiste essentiellement en deux conduites d'évacuation (6a, 6b) qui conduisent depuis deux dispositifs de couplage (17a, 17b) qui sont adaptées pour le raccordement du système extracorporel de conduite de sang (5), à un segment de conduite d'évacuation (6c) dans lequel elles se réunissent et de là, à la conduite de sortie liquide de dialyse (21) et au réservoir de déchets (39), et deux conduites d'amenée de liquide de substitution (44a, 44b) dotées respectivement d'un dispositif de couplage (18a, 18b) pour le raccordement du système extracorporel de conduite de sang (5).

2. Machine de dialyse selon la revendication 1, **caractérisée en ce que** les deux conduites d'évacuation (6a, 6b) sont reliées l'une à l'autre selon la technique des fluides.

3. Machine de dialyse selon l'une quelconque des revendications précédentes, **caractérisée en ce que** la conduite d'amenée de liquide de substitution (44, 44a, 44b) est reliée à la conduite d'évacuation (6, 6a, 6b) selon la technique des fluides.

4. Machine de dialyse selon l'une quelconque des revendications précédentes, **caractérisée en ce que** dans la conduite d'évacuation (6, 6a, 6b) est disposé un séparateur d'air (43).

5. Machine de dialyse selon l'une quelconque des revendications précédentes, **caractérisée en ce que** la pompe de liquide de substitution (30) est disposée en amont d'une ramification par rapport aux dispositifs de couplage (17a, 17b, 18a, 18b) et en aval de la ramification dans chaque conduite d'amenée de liquide de substitution (44a, 44b), une vanne de commande (31, 32) est utilisée pour la commande sélective d'une pré-et/ou post-dilution.
